# EUROPEAN PATENT SPECIFICATION

(11) **EP 0 768 292 B1**
(45) Date of publication and mention of the grant of the patent: **12.01.2000**
(21) Application number: 96202754.6
(22) Date of filing: 03.10.1996
(51) Int. Cl.: C07C 45/53, C07C 45/33, C07C 409/06, C07C 29/132, C07C 29/50

(54) **Process for decomposing cycloalkyl hydroperoxide**
Verfahren zur Zersetzung von Cycloalkylhydroperoxid
Procédé pour la décomposition de l'hydroperoxyde de cycloalkyle

(30) Priority: 13.10.1995 BE 9500853
(43) Date of publication of application: 16.04.1997
(73) Proprietor: DSM N.V., 6411 TE Heerlen (NL)
(72) Inventor: Kragten, Ubladus Franciscus, 6191 EK Beek (NL); Baur, Henricus Anna Christiaan, 6049 BE Roermond (NL); Housmans, Johannes Gerardus Hubertus Maria, 6051 HE Maasbracht (NL)

(56) References cited:
- EP-A- 0 004 105
- EP-A- 0 092 867
- EP-A- 0 659 726
- FR-A- 2 140 088

## Description

The invention relates to a process for decomposing a mixture comprising cycloalkyl hydroperoxide in the presence of an alkali metal hydroxide dissolved in an aqueous phase.

Such a process is already known from EP-A-4105, which discloses that the decomposition of cycloalkyl hydroperoxide is carried out in the presence of, particularly, sodium hydroxide. Although high conversions to cycloalkanones and cycloalkanols are achieved, the reaction velocity is relatively low.

The reaction velocity constant, a measure of the reaction velocity, is an important parameter to increase. The higher this constant, the more efficient the decomposition reaction. In many cases this also implies that side reactions take place to a lesser degree. In addition, the decomposition reaction can be carried out in a smaller reactor, which means a lower investment, or in an existing reactor more decomposition products, i.e. cycloalkanones and cycloalkanols, can be formed. The cycloalkanones and cycloalkanols can be used in the preparation of ε-caprolactam, which in turn can be used as a raw material in the production of nylons.

The object of the invention is to provide a process having a greater reaction velocity for decomposing cycloalkyl hydroperoxide into the desired products cycloalkanol/cycloalkanone.

This object is achieved in that, besides the alkali metal hydroxide, also at least 10 wt.%, relative to the aqueous phase, of one or more alkali metal salts is present.

The alkali metal salts that can be used for this purpose are preferably soluble alkali metal salts.Suitable salts are alkali metal carbonates, and in particular alkali metal carboxylates. Alkali metal salts of mono- and polycarboxylic acids in which the carboxylic acid moiety preferably comprises 1-24 C-atoms are suitable; more preferably the carboxylic acid moiety comprises 1-12 C-atoms. Very suitable as alkali metal are sodium and potassium. Preferably, the alkali metal is sodium. Examples of suitable carboxylic acids are acetic acid, propionic acid, butyric acid, adipic acid, hexanoic acid, pentanoic acid, propane dicarboxylic acid, hexane dicarboxylic acid, stearic acid and decanoic acid. Special preference is given to the use of mixtures of different carboxylic acids, since these are simply obtainable.

The alkali metal salts are used in a quantity of at least 10 wt.% based on the aqueous phase that is present besides the organic phase containing the cycloalkyl hydroperoxide. The wt.% is calculated on the basis of the alkali metal salt. The salt concentration is preferably higher than 15 wt.%. The salt concentration is preferably lower than 35 wt.%. More preferably, use is made of a salt concentration between 20 and 35 wt.% calculated on the basis of the total salts. It is also possible to use a higher salt concentration, for instance 45 wt.%. However, this has the disadvantage that crystallization of the metal carboxylates may occur on cooling of this process stream. This can be prevented by diluting this process stream.

The mixture containing cycloalkyl hydroperoxide can be obtained by oxidation of a cycloalkane with 5-12 C-atoms in the ring f.e. in the liquid phase with an oxygen-containing gas. As cycloalkane use is preferably made of cyclopentane, cyclooctane, cyclododecane and in particular cyclohexane. The oxidation mixture formed may contain other peroxides besides the cycloalkyl hydroperoxide, for instance dicycloalkyl peroxide.

The oxidation usually takes place in the liquid phase. As oxygen-containing gas use can be made for instance of air or pure oxygen. Suitable oxidation temperatures are between 120 and 200°C. Preferably, a temperature between 140 and 190°C is used.

The oxidation reaction is carried out for 5 minutes to 24 hours. The pressure must be such that a liquid phase is maintained in the system. The pressure is usually between 0.3 and 5 MPa, preferably between 0.4 and 2.5 MPa.

Preferably, the oxidation is operated continuously and preferably takes place in a system of series-arranged reactors or a compartmentalized tubular reactor. Usually the reaction is operated autothermally, or via temperature control. Temperature control usually takes place by discharging the reaction heat via a gas stream, intermediate cooling or using other methods known to one skilled in the art. To prevent transition metals (which promote the decomposition of cycloalkyl hydroperoxide) entering the mixture to be oxidized, preferably reactors with inert internal walls are chosen. For instance, use can be made of reactors with internal walls made of passivated steel, aluminium, tantalum, glass or enamel. This is important especially for small production capacities, in which case the ratio between wall area and liquid volume is unfavourable. For large capacities, a reactor with inert internal walls is not required. It should be clear that, if a negligible quantity of metal ions enters the oxidation mixture, this does not have any substantial effect on the reaction, and in the context of this invention non-catalyzed cycloalkane oxidation may be used.

In contrast to the non-catalyzed cycloalkane oxidation, the catalyzed oxidation by a metal such as cobalt and chromium produces a reaction mixture with a relatively small quantity of cycloalkyl hydroperoxide compared with the quantity of cycloalkanone and cycloalkanol produced. Notwithstanding this, the process according to the invention is also advantageous in catalyzed oxidation where only a small quantity of cycloalkyl hydroperoxide remains.

Usually the product of the uncatalyzed oxidation of cyclohexane contains at least comparable quantities, in wt.%, of cyclohexyl hydroperoxide and of cyclohexanol + cyclohexanone. Often, the mixture after the oxidation reaction contains a quantity of cyclohexyl hydroperoxide that is more than 2 times the quantity of cyclohexanol + cyclohexanone. In contrast, the catalyzed oxidation produces a mixture which contains less than 50% cyclohexyl hydroperoxide compared with the weight percentage of cyclohexanol + cyclohexanone. Often, there is even less than 40% cycloalkyl hydroperoxide compared with the weight percentage of cyclohexanol + cyclohexanone.

The cycloalkyl hydroperoxide concentration in the reaction mixture as it leaves the (last) oxidation reactor is generally between 0.1 and 8.0 wt.% The cycloalkanol concentration in this mixture is generally between 0.1 and 10 wt.%. The cycloalkane conversion is generally between 0.5 and 25 wt.%. The cyclohexane conversion is generally between 2 and 6 wt.%.

For the decomposition of cycloalkyl hydroperoxide so much alkali metal hydroxide is added that the concentration of OH⁻, ([OH⁻]) of the water phase on completion of the decomposition is at least 0.1 N, preferably at least 0.6 N. In principle, an [OH⁻] higher than 2 N is possible, but this does not offer any advantages. Such a high concentration might result in side-reactions occurring, for instance aldol condensation of cycloalkanone. The quantity of alkali metal hydroxide used is therefore preferably such that the [OH⁻] in the aqueous phase upon completion is between about 0.1 N and about 2 N. In particular, such a quantity of hydroxide is used that an [OH⁻] between 0.6 and 1 N is obtained. The most suitable alkali metal hydroxides for the process according to the invention are sodium hydroxide and potassium hydroxide.

The decomposition reaction is preferably carried out in the presence of at least one catalyst, a cycloalkyl hydroperoxide decomposition-promoting metal salt. This is generally a salt of a transition metal. Examples of suitable transition metals are cobalt, chromium, manganese, iron, nickel, copper, or mixtures of these, such as for instance a mixture of cobalt and chromium. Preferably, the transition metal salt is water soluble. Metal sulphates and metal acetates have proved to be very suitable salts. The quantity of transition metal salt may be 0.1 - 1000 ppm, calculated as metal, relative to the weight of the aqueous phase. However, it is also possible to use larger quantities of transition metal salt. Preferably, use is made of 0.1 - 10 ppm of metal. The transition metal salt can be added, optionally in combination with the alkali metal hydroxide, as an aqueous solution to the mixture containing the cycloalkyl hydroperoxide. It is also possible to add the transition metal as an organic salt, dissolved in an organic solvent, to the reaction mixture. For example, the cycloalkane corresponding to the cycloalkyl hydroperoxide may be used as an organic solvent.

The decomposition reaction takes place by allowing the cycloalkyl hydroperoxide containing mixture to react for 5 to 300 minutes. Preferably, the residence time in the decomposition reactor is 15 to 120 minutes, but the time needed can simply be determined by one skilled in the art.

The decomposition reaction preferably takes place in a stirred tank reactor, and with special preference in a countercurrent column equipped with stirring gear.

To achieve an efficient cycloalkyl hydroperoxide decomposition, the volume ratio between the aqueous phase and the organic phase in the decomposition reactor is preferably higher than 0.02. Preferably, a ratio of 0.05 - 0.25 is used. However, these volume ratios are not critical and can, if desired, be adjusted by one skilled in the art.

The cycloalkyl hydroperoxide decomposition can take place at a temperature between 60 and 180°C. Preferably, the decomposition takes place at a temperature between 60 and 100°C.

The decomposition reaction can be carried out either at atmospherical or at elevated pressure. The decomposition of cycloalkyl hydroperoxide can advantageously be carried out at a pressure that is of the same order as the pressure used for oxidation of the corresponding cycloalkane; however, it may also be advantageous to evaporate part of the cycloalkane after oxidation by allowing a pressure reduction to take place (flashing). The pressure during the decomposition reaction is then preferably about 0.1-0.6 MPa, more in particular the decomposition reaction is carried out at atmospherical pressure.

After the decomposition the aqueous phase can be separated from the organic phase. The organic phase can then be washed to remove trace residues of salt contained in the aqueous phase. The aqueous phase can be reused in the decomposition reaction. In that case the aqueous phase already contains alkali metal salts of mono- or polycarboxylic acids. The carboxylic acids can be formed as by-product in the oxidation or in the decomposition, upon which, owing to the presence of alkali metal, a salt is formed with the carboxylic acid. Reuse of the aqueous phase has the advantage that the ratio between aqueous phase and organic phase can be set and monitored in a simple manner.

Distillation of the organic phase eventually yields a mixture of cycloalkanone and cycloalkanol.

The decomposition reaction can be carried out both continuously and batchwise.

### Example I

At a temperature of 70°C, 107 ml of an aqueous phase containing dissolved NaOH (1500 mmol NaOH/kg) and sodium acetate (15 wt.%) was added to 250 ml of a cyclohexane oxidation mixture containing, per kilogramme, 190 mmol cyclohexyl hydroperoxide (CHHP), 40 mmol cyclohexanone (ON) and 90 mmol cyclohexanol (OL). Decomposition of the CHHP was monitored by means of a iodometric titration. The first-order velocity constant was 11 * 10⁻³ min⁻¹.

### Comparative experiment A

Example I was repeated, this time 107 ml of an aqueous phase containing dissolved NaOH (1500 mmol NaOH/kg) being added. Decomposition of the CHHP was monitored by means of a iodometric titration. The first-order velocity constant was 8 * 10⁻³ min⁻¹.

### Example II

At a temperature of 70°C, 107 ml of an aqueous phase containing dissolved NaOH (1500 mmol NaOH/kg), sodium acetate (15 wt.%) and Cr(NO₃)₃ (10 ppm Cr) was added to 250 ml of a cyclohexane oxidation mixture containing, per kilogramme, 190 mmol cyclohexyl hydroperoxide (CHHP), 40 mmol cyclohexanone (ON) and 90 mmol cyclohexanol (OL). Decomposition of the CHHP was monitored by means of a iodometric titration. The first-order velocity constant was 13 * 10⁻³ min⁻¹.

### Comparative experiment B

Example II was repeated, this time 107 ml of an aqueous phase containing dissolved NaOH (1500 mmol NaOH/kg) and Cr (NO₃)₃ (10 ppm Cr) being added. Decomposition of the CHHP was monitored by means of a iodometric titration. The first-order velocity constant was 8 * 10⁻³ min⁻¹.

### Continuous experiments in a glass reactor

### Procedure

The set-up consisted of two series-arranged, double-walled glass reactors with a liquid volume per reactor of 500 ml. Both were provided with baffles, stirrer, reflux cooler and an overflow. Fresh cyclohexane oxidation mixture and fresh aqueous phase were introduced through the first reactor. The temperature in both reactors was controlled by means of two independent thermostats.

### Example III

The first reactor was fed with 17.0 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 1.95 ml/min of an aqueous phase was added which contained dissolved NaOH (750 mmol NaOH/kg), Na₂CO₃ (354 mmol/kg), CoSO₄ (4.3 ppm Co) and a mixture of sodium salts of mono- and dicarboxylic acids (C₁ through C₆) (20 wt.% in water). Decomposition of the CHHP took place at a temperature of 69°C in the first reactor and a temperature of 66°C in the second reactor. Decomposition of the CHHP was monitored by means of a iodometric titration. The first-order velocity constant, calculated over both reactors, was 140 * 10⁻³ min⁻¹. The CHHP conversion exceeded 95%.

### Example IV

Example III was repeated, this time the first reactor being fed with 16.7 ml/min of a cyclohexane oxidation mixture containing, per kilogramme, 182 mmol cyclohexyl hydroperoxide (CHHP), 42 mmol cyclohexanone (ON) and 86 mmol cyclohexanol (OL). In addition, 1.93 ml/min of an aqueous phase was added which contained dissolved NaOH (750 mmol NaOH/kg), Na₂CO₃) (365 mmol/kg Na₂CO₃, CoSO₄ (4.3 ppm Co) and a mixture of sodium salts of mono- and dicarboxylic acids (C₁ through C₆) (20 wt.% in water). Decomposition of the CHHP took place at a temperature of 67°C in the first reactor and a temperature of 66°C in the second reactor. Decomposition of the CHHP was monitored by means of a iodometric titration. The first-order velocity constant, calculated over both reactors, was 131 * 10⁻³ min⁻¹. The CHHP conversion exceeded 95%.

### Example V

Example III was repeated, this time the first reactor being fed with 16.9 ml/min of a cyclohexane oxidation mixture containing, per kilogramme, 182 mmol cyclohexyl hydroperoxide (CHHP), 42 mmol cyclohexanone (ON) and 86 mmol cyclohexanol (OL). In addition, 1.90 ml/min of an aqueous phase was added which contained dissolved NaOH (750 mmol NaOH/kg), Na₂CO₃ (375 mmol/kg, CoSO₄ (4.3 ppm Co) and a mixture of sodium salts of mono- and dicarboxylic acids (C₁ through C₆) (15 wt.% in water). Decomposition of the CHHP took place at a temperature of 67°C in the first reactor and a temperature of 66°C in the second reactor. Decomposition of the CHHP was monitored by means of a iodometric titration. The first-order velocity constant, calculated over both reactors, was 110 * 10⁻³ min⁻¹. The CHHP conversion exceeded 93%.

### Comparative experiment C

Example III was repeated, the first reactor being fed with 16.6 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 1.94 ml/min of an aqueous phase was added which contained dissolved NaOH (750 mmol NaOH/kg), Na₂CO₃ (365 mmol/kg) and CoSO₄ (4.3 ppm Co). Decomposition of the CHHP took place at a temperature of 69°C in the first reactor and a temperature of 66°C in the second reactor. Decomposition of the CHHP was monitored by means of a iodometric titration. The first-order velocity constant, calculated over both reactors, was 60 * 10⁻³ min⁻¹. The CHHP conversion was lower than 87%.

### Continuous experiments at elevated pressure

### Procedure

The set-up consisted of a Cr/Ni steel reactor with a liquid volume of 1000 ml. The reactor was provided with baffles, stirrer, reflux cooler and an overflow. Fresh cyclohexane oxidation mixture and fresh aqueous phase were introduced by two independent pumps. The temperature in the reactor was controlled by means of a thermostat.

### Example VI

The reactor was fed with 75.3 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 15.6 ml/min of an aqueous phase was added which contained dissolved NaOH (625 mmol NaOH/kg), Na₂CO₃ (445 mmol/kg), CoSO₄ (10 ppm Co) and a mixture of sodium salts of mono- and dicarboxylic acids (C₁ through C₆) (15 wt.% in water). Decomposition of the CHHP took place at a temperature of 85°C. Decomposition of the CHHP was monitored by means of a iodometric titration. The first-order velocity constant was 1.97 min⁻¹ (2.63 min⁻¹ at 90°C). The CHHP conversion exceeded 95%.

### Example VII

The reactor was fed with 75.3 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 15.6 ml/min of an aqueous phase was added which contained dissolved NaOH (935 mmol NaOH/kg), CoSO₄ (10 ppm Co) and sodium acetate (12 wt.% in water). Decomposition of the CHHP took place at a temperature of 105°C. Decomposition of the CHHP was monitored by means of a iodometric titration. The first-order velocity constant was 5.65 min⁻¹ (2.49 min⁻¹ at 90°C). The CHHP conversion exceeded 98%.

### Example VIII

The reactor was fed with 76.5 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 15.3 ml/min of an aqueous phase was added which contained dissolved NaOH (750 mmol NaOH/kg), Na₂CO₃ (315 mmol/kg), CoSO₄ (10 ppm Co) and a mixture of sodium salts of mono- and dicarboxylic acids (C₁ through C₆) (25 wt.% in water). Decomposition of the CHHP took place at a temperature of 85°C. Decomposition of the CHHP was monitored by means of a iodometric titration. The first-order velocity constant was 1.78 min⁻¹ (2.37 min⁻¹ at 90°C). The CHHP conversion exceeded 95%.

### Comparative experiment D

The reactor was fed with 74.5 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 15.0 ml/min of an aqueous phase was added which contained dissolved NaOH (660 mmol NaOH/kg), Na₂CO₃ (420 mmol/kg) and CoSO₄ (10 ppm Co). Decomposition of the CHHP took place at a temperature of 96°C. Decomposition of the CHHP was monitored by means of a iodometric titration. The first-order velocity constant was 0.45 min⁻¹ (0.32 min⁻¹ at 90°C). The CHHP conversion was lower than 85%.

### Comparative experiment E

The reactor was fed with 74.5 ml/min of a cyclohexane oxidation mixture containing, per kilogramme, 153 mmol cyclohexyl hydroperoxide (CHHP), 53 mmol cyclohexanone (ON) and 105 mmol cyclohexanol (OL). In addition, 15.0 ml/min of an aqueous phase was added which contained dissolved NaOH (1600 mmol NaOH/kg), Na₂CO₃ (630 mmol/kg) and CoSO₄ (10 ppm Co). Decomposition of the CHHP took place at a temperature of 95°C. Decomposition of the CHHP was monitored by means of a iodometric titration. The first-order velocity constant was 0.97 min⁻¹ (0.73 min⁻¹ at 90°C). The CHHP conversion was lower than 92%.

## Claims

1. Process for decomposing a mixture comprising cycloalkyl hydroperoxide in the presence of an alkali metal hydroxide dissolved in an aqueous phase, characterized in that, besides the alkali metal hydroxide, at least 10 wt.%, relative to the aqueous phase, of one or more alkali metal salts is present.

2. Process according to claim 1, characterized in that the alkali metal salts are alkali metal carbonates or alkali metal salts of mono- and polycarboxylic acids, the carboxylic acid moiety of the monocarboxylic acid or the polycarboxylic acid containing 1-24 C-atoms.

3. Process according to either of claims 1 or 2, characterized in that the alkali metal is sodium or potassium.

4. Process according to any one of claims 1-3, characterized in that the salt concentration is lower than 45 wt.%, based on the aqueous phase that is present besides the phase containing cycloalkyl hydroperoxide.

5. Process according to any one of claims 1-4, characterized in that the salt concentration is 20-35 wt.%.

6. Process according to any one of claims 1-5, characterized in that such a quantity of alkali metal hydroxide is used that at the end of the decomposition reaction a concentration of OH⁻ of between 0.1 and 2 N is present.

7. Process according to any one of claims 1-6, characterized in that during the decomposition also between 0.1 and 1000 ppm of a decomposition-promoting salt of a transition metal is present.

8. Process according to any one of claims 1-7, characterized in that the mixture containing cycloalkyl hydroperoxide has been obtained by oxidation of a corresponding cycloalkane at a temperature between 120 and 200°C, a pressure between 0.3 and 5 MPa, in the absence of an oxidation catalyst.

## Patentansprüche

1. Verfahren zum Zersetzen einer Mischung, umfassend Cycloalkylhydroperoxid in der Gegenwart eines in einer wäßrigen Phase gelösten Alkalimetallhydroxids, dadurch gekennzeichnet, daß neben dem Alkalimetallhydroxid wenigstens 10 Gew.-% in bezug auf die wäßrige Phase von einem oder mehreren Alkalimetallsalzen vorhanden sind.

2. Verfahren nach Anspruch 1, dadurch gekennzeichnet, daß die Alkalimetallsalze Alkalimetallcarbonate oder Alkalimetallsalze von Mono- und Polycarbonsäuren sind, wobei der Carbonsäurerest der Monocarbonsäure oder der Polycarbonsäure 1 bis 24 Kohlenstoffatome enthält.

3. Verfahren nach einem der Ansprüche 1 oder 2, dadurch gekennzeichnet, daß das Alkalimetall Natrium oder Kalium ist.

4. Verfahren nach einem der Ansprüche 1 bis 3, dadurch gekennzeichnet, daß die Salzkonzentration niedriger als 45 Gew.-% basierend auf der wäßrigen Phase, welche neben der Cycloalkylhydroperoxid enthaltenden Phase vorhanden ist, ist.

5. Verfahren nach einem der Ansprüche 1 bis 4, dadurch gekennzeichnet, daß die Salzkonzentration 20 bis 35 Gew.-% beträgt.

6. Verfahren nach einem der Ansprüche 1 bis 5, dadurch gekennzeichnet, daß eine derartige Menge an Alkalimetallhydroxid verwendet wird, daß am Ende der Zersetzungsreaktion eine OH⁻-Konzentration zwischen 0,1 und 2 N vorhanden ist.

7. Verfahren nach einem der Ansprüche 1 bis 6, dadurch gekennzeichnet, daß während der Zersetzung auch zwischen 0,1 und 1000 ppm eines die Zersetzung fördernden Salzes eines Übergangsmetalls vorhanden sind.

8. Verfahren nach einem der Ansprüche 1 bis 7, dadurch gekennzeichnet, daß die Cycloalkylhydroperoxid enthaltende Mischung durch Oxidation eines entsprechenden Cycloalkans bei einer Temperatur zwischen 120 und 200 °C, einem Druck zwischen 0,3 und 5 MPa in Abwesenheit eines Oxidationskatalysators erhalten wurde.

## Revendications

1. Procédé de décomposition d'un mélange comprenant de l'hydroperoxyde de cycloalkyle en présence d'un hydroxyde de métal alcalin dissous dans une phase aqueuse, caractérisé en ce qu'en plus de l'hydroxyde de métal alcalin, au moins 10% en poids, par rapport à la phase aqueuse, d'un ou plusieurs sels de métaux alcalins sont présents.

2. Procédé selon la revendication 1, caractérisé en ce que les sels de métaux alcalins sont des carbonates de métaux alcalins ou des sels de métaux alcalins d'acides mono et polycarboxyliques, le fragment acide carboxylique de l'acide monocarboxylique ou de l'acide polycarboxylique contenant 1 à 24 atomes de carbone.

3. Procédé selon la revendication 1 ou 2, caractérisé en ce que le métal alcalin est le sodium ou le potassium.

4. Procédé selon l'une quelconque des revendications 1 à 3, caractérisé en ce que la concentration en sel est inférieure à 45% en poids, basé sur la phase aqueuse présente en plus de la phase contenant l'hydroperoxyde de cycloalkyle.

5. Procédé selon l'une quelconque des revendications 1 à 4, caractérisé en ce que la concentration en sel est 20 à 35% en poids.

6. Procédé selon l'une quelconque des revendications 1 à 5, caractérisé en ce qu'on utilise une quantité d'hydroxyde de métal alcalin telle qu'à la fin de la réaction de décomposition, une concentration en OH⁻ entre 0,1 et 2 N est présente.

7. Procédé selon l'une quelconque des revendications 1 à 6, caractérisé en ce que pendant la décomposition, est également présent, entre 0,1 et 1000 ppm, un sel d'un métal de transition activant la décomposition.

8. Procédé selon l'une quelconque des revendications 1 à 7, caractérisé en ce que le mélange contenant l'hydroperoxyde de cycloalkyle est obtenu par oxydation d'un cycloalcane correspondant à une température entre 120 et 200°C, une pression entre 0,3 et 5 MPa, en l'absence d'un catalyseur d'oxydation.
